# EUROPEAN PATENT SPECIFICATION

(11) **EP 0 478 692 B1**
(45) Date of publication and mention of the grant of the patent: **24.01.1996**
(21) Application number: 90911032.2
(22) Date of filing: 20.06.1990
(51) Int. Cl.: A61M 1/14, A61M 1/34

(54) **HEMODIALYSIS SYSTEM**
HEMODIALYSESYSTEM
SYSTEME D'HEMODIALYSE

(30) Priority: 20.06.1989 US 368665
(43) Date of publication of application: 08.04.1992
(73) Proprietor: THE BOARD OF REGENTS OF THE UNIVERSITY OF WASHINGTON, Seattle, Washington 98195 (US)
(72) Inventor: AHMAD, Suhail, Seattle, WA 98125 (US); COLE, James, J., Arlington, WA 98223 (US); JENSEN, William P., Seattle, WA 98155 (US)
(74) Representative: Grünecker, Kinkeldey, Stockmair & Schwanhäusser Anwaltssozietät
(86) International application number: US9003467
(87) International publication number: WO9015631

(56) References cited:
- EP-A- 0 263 384
- DE-A- 2 513 489
- DE-A- 2 559 241
- DE-A- 3 307 830
- DE-A- 3 720 664
- DE-C- 3 444 671

## Description

The present invention relates to hemodialysis systems, and more particularly, to an improved such system having blood and hemofiltrate circuits interconnected at a hemofilter and an air collection chamber, and having a novel blood pump operating according to the patient's blood delivery rate.

### Background of the Invention

Hemodialysis treatment is employed as a therapeutic measure then a patient's kidneys no longer perform their blood purifying function because of disease or traumatic removal. Kidney failure results in the accumulation of toxic waste in the patient's blood and eventual death from uremic poisoning, unless the waste material is removed by some artificial means. In hemodialysis of the type to which the present invention relates, the patient's blood is circulated from the patient in a closed blood circuit by a pump to one side of a membrane contained within a hemodialyzer (i.e., artificial kidney). The membrane has pores of microscopic size through which waste products from the blood pass. The pores are, however, too small to permit blood cells and proteins to leave the body. A dialysis fluid (dialysate) is circulated on the other side of the hemodialyzer membrane to remove the baste products. The dialyzed blood is returned to the patient.

The blood pump normally used in contemporary dialysis systems is a roller pump in which rollers on rotating arms radiating from a motor-driven shaft progressively sneeze closed a section of flexible tubing in the circuit connecting the patent's blood system to the hemodialyzer. The speed of the pump motor is set to give a pump output in accordance with the anticipated patient's blood delivery rate. However, this delivery rate will normally vary during treatment and may drop below the pump output setting, a condition likely to collapse and obstruct the connection tubing. In contemporary dialysis machines an alarm is triggered and the blood pump is stopped if the patient's blood delivery rate drops below the pump flow rate setting. To minimize the occurrence of such a condition the blood pump is normally given a flow rate setting below the anticipated blood flow rate from the patient to allow for downward fluctuations of the patient's blood delivery rate. Hence, the treatment time is longer than would be necessary if the pumping rate continuously matched the patient's blood delivery rate.

A hemodialysis system according to the first part of claim 1 is known from DE-A-25 13 489.

The system according to claim 1 regulates the blood flow rate through the blood circuit of the dialysis system according to the available blood delivery rate from the patient, thereby avoiding recurring alarms and reducing staffing needs. In some instances, optimization of blood flow may also shorten dialysis treatment time. Regulation by the blood delivery rate of the patient is accomplished in the practice of the present invention by providing a blood pump having a compressible pump chamber and having flexible ingress and egress tubes at opposite ends of the chamber which extend through pinching devices functioning together with the tubes as ingress and egress pinch valves. The pump chamber walls are sufficiently elastic to self return the pump chamber to its normal size from a compressed condition when compression is released.

Expansion of the pump chamber toward its normal size from a compressed empty condition creates a suction which aids in filling the chamber with blood from the patient. When filling of the pump chamber is complete the ingress valve closes and the egress valve opens. Then compression of the pump chamber commences for delivery of blood from the chamber to the rest of the dialysis system during a compression stroke. At the close of the chamber compression stroke the compression device commences a chamber expansion stroke with the egress valve closed and the ingress valve open. During this chamber expansion stroke the compressing device remains in engagement with a wall of the pump chamber, and the chamber expansion stroke is permitted to advance only at a pace determined by the blood delivery rate from the patient as it then exists.

In contemporary dialysis systems usually the tubing is replaced after each treatment. This is always done in dialysis treatment centers in which multiple patients use the same dialysis machine. The time and expense involved in tubing replacement is significant. The inventive system makes it possible to backwash the tubing and other components of the dialysis system so as to make it possible to reuse the system with the tubing remaining in place on the machine for several treatments in situations such as home dialysis in which the dialysis machine is only used by one patient. The present invention also makes it possible in installations in which a dialysis machine is used for treating multiple patients in sequence to remove the tubing and system elements to which the tubing is connected as a module for reuse for the same patient.

Occasionally a patient will experience discomfort or shock symptoms. In the past such occurrences have been remedied by an infusion of a sterile solution from an external source to the returning blood. The inventive system provides a filtrate circuit integrated in a closed system with the blood circuit in the dialysis machine in such a manner that some of the filtrate can readily be pumped back into the blood circuit and returned to the patient rather than supplementing with fluids from an external source.

### Brief Description of the Drawings

Figure 1 is a schematic of a hemodialysis system embodying the present invention.

Figure 2 is a schematic of a valve block for use during post-treatment operations on the system.

Figure 3 is a layout of the pumping portion of the system when a single pumping chamber is used, and showing the condition when the chamber is slightly compressed.

Figure 4 is a layout showing use of a two-chamber pumping arrangement in the system, and illustrating the condition when one chamber is nearly fully compressed and the other chamber is nearly fully expanded.

### Detailed Description of the Invention

In accordance with the present invention a blood circuit which includes a supply-regulated reversible pump 18 with ingress and egress pinch valves 19-20, the blood side of a hemofilter 22, a hemodialyzer 24, and an air-collection chamber 26 arranged in series, interacts in a closed system with a filtrate circuit comprising the filtrate side of the hemofilter 22, a reversible roller pump 28, a reservoir 30, and pinch valves 32, 33. A suitable anticoagulant such as heparin is introduced and metered to the blood circuit by a syringe pump 34 via a tube 35 which preferably discharges to the air-collection chamber 26.

When a patient is expected to be on dialysis for an extended period of time it is common to surgically create for ease of blood access an enlarged vein in one of the patient's limbs. In preparation for a dialysis treatment, two needles are inserted a few centimeters apart into the blood access. The upstream needle is connected to a flexible tube 36 (hereinafter called the "arterial tube") leading to the pump 18, and the downstream needle is connected to a flexible tube 37 (hereinafter called the "venous tube") returning to the patient from the air-collection chamber 26. In some instances a single needle is used, in which case the arterial and venous tubes 36, 37 are both connected to the needle via a T-fitting.

The tubes 36, 37 are part of the blood circuit together with a flexible tube 38 between the pump 18 and hemofilter 22, tube 39 between the hemofilter 22 and dialyzer 24, and tube 40 connecting the dialyser to the air-collection chamber 26. The tubing in the filtrate system comprises a flexible tube 41 connected to the filtrate side of the hemofilter 22, a flexible tube 42 connected to the top of the air-collection chamber 26, and a flexible tube 43 leading from the bottom of the reservoir 30 to a Y-connection with the tubes 41, 42. The reservoir 30 may comprise a vessel with a filtered air vent or a collapsible air-tight bag. It is also preferred to provide a tube 44 extending from the pump end of the tube 38 to a connection with the tube 43 and to equip the tube 44 with a pinch valve 46. The tubes 41, 42, and 44 preferably have branches containing pressure sensors 45-47, respectively, for pressure monitoring. The dialysate side of the dialyzer 24 has supply and discharge ports 48, 49 connected to a dialysate supply and to a drain.

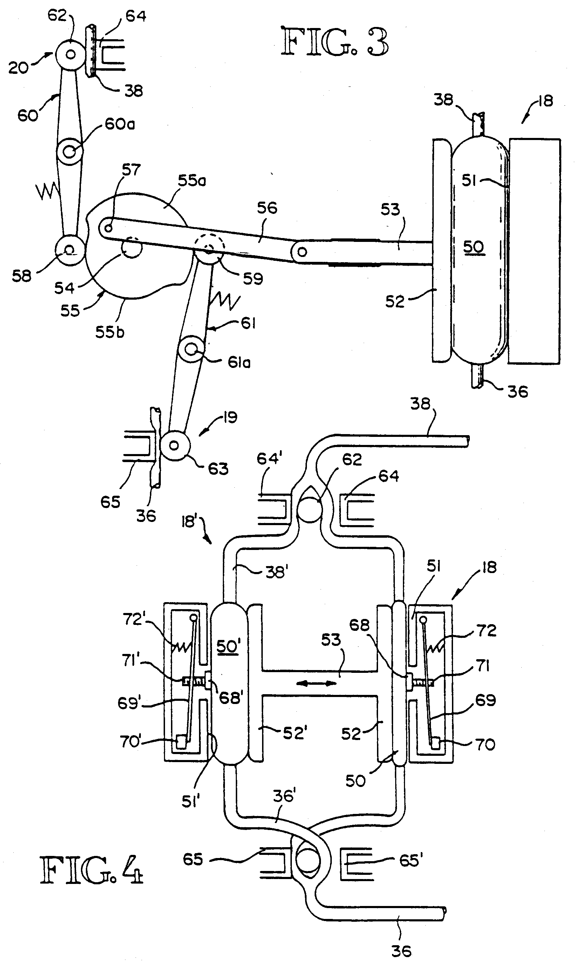

Directing attention to Figure 3, the blood pump 18 has an elongated squeeze chamber 50 functioning between pinch valves 19-20 acting on the arterial tube 36 and on the egress tube 38 leading to the hemofilter 22. The squeeze chamber 50 is located between a stationary wall 51 and a reciprocating compression shoe 52. The shoe 52 is mounted on a slide rod 53 which is slowly driven back and forth by a driven shaft 54 via a combination crank and cam member 55 and a connecting link 56 which is pivotally connected at its ends by pins 57 to member 55 and slide rod 53. A reversible motor with a gear reduction unit (not shown) may be used to drive the shaft 54.

The member 55 has a lifting lobe 55a which encompasses about 190 degrees and a return section 55b. Riding on the periphery of member 55 are two diametrically opposite cam followers 58, 59 mounted on the ends of rockers 60, 61. These are centrally pivotally mounted at 60a, 61a and are spring loaded to swing the cam followers into constant engagement with member 55. On their ends opposite from the cam followers 58, 59 the rockers 60, 61 carry round pinch elements 62, 63 which oppose stationary blocks 64, 65. The pinch element 63 and block 65 comprise the ingress valve 19, and the pinch element 63 and block 64 comprise the egress valve 20. The arterial ingress tube 36 passes between the pinch element 63 and the block 65, and the egress tube 38 passes between the pinch element 62 and the block 64. When the cam followers 58, 59 are lifted radially away from the shaft 54 by the lobe 55a as the shaft 54 turns clockwise, for example, the pinch elements 62, 63 are urged toward the blocks 64, 65 and cooperate therewith to respectively pinch the tubes 38, 36 to a closed position. When the cam followers 58, 59 return to engagement with the return section 55b, the pinch elements 62, 63 are moved out of tube pinching position, thereby opening tubes 38 and 36, respectively.

The stationary wall 51 is interrupted to expose a projecting button 68 extending from a swing am 69 which is arranged to engage a microswitch 70 when the swing arm 69 is swung away from the compression chamber 50 responsive to engagement of the button 68 by the opposing wall of the chamber 50. The button 68 is preferably connected to the swing arm 69 by an adjusting screw 71 so that the extent of engagement of the chamber wall necessary to operate the microswitch 70 can be readily adjusted. A spring 72 biases the swing arm 69 toward the chamber 50. The microswitch 70 is wired in series with the motor driving the shaft 54 so that the motor operates only when the microswitch is closed.

As will later be discussed, normally at the start of a dialysis treatment with the present invention, the system will be filled with sterile dialysate which is not harmful to the patient if introduced to the patient. As previously indicated, the patient is commonly connected to the arterial and venous tubes 36, 37 via needles inserted through the wall of a blood access vein. Dialysis is normally commenced with the ingress valve 19 of the blood pump open and the egress valve 20 thereof closed. As the vessel 50 expands due to its own elasticity, it creates sufficient suction acting in conjunction with the pressure of the blood exiting the patient, to potentially fill the vessel 50.

When blood initially enters the squeeze chamber 50 from the patient, normally the microswitch 70 is open and the motor driven shaft 54 is stationary. Then as the chamber 50 expands and begins to fill with blood; it firmly engages the compression shoe 52, wall 51, and button 68. The microswitch 70 is responsively closed, thereby starting the motor to drive shaft 54 so that expansion of the chamber 50 can continue as entry flow of the blood from the patient continues. If the shaft 54 turns so rapidly that the slide rod 53 moves away from the stationary wall 51 at a rate faster than the expansion rate of the squeeze chamber 50, the microswitch 70 will open, thereby stopping the motor until the expansion of the chamber 50 catches up to the then position of the compression shoe 52. Thus the blood pump 18 is regulated by the supply of blood from the patient. Filling of the chamber 50 continues until the cam follower 59 begins to climb down from the lobe 55a to close the ingress valve 19 and, the cam follower 58 reaches the lobe 55a to open the egress valve 20 after the ingress valve is closed. By this time, the connecting link 56 has begun to reverse its stroke to commence compression of the chamber 50 and the start of a pumping cycle. Compression continues until the start of another chamber filling cycle.

When the egress valve 20 opens at the start of a pumping cycle, and blood is forced from the vessel 50 by the compressing action of the shoe 52, the pinch valve 32 in the filtrate circuit is opened and the filtrate pump 28 is started so that when the pumped blood passes through the hemofilter 22, filtrate from the blood will be pumped from the hemofilter to the reservoir 30 via tubes 41 and 43. The circulating blood then passes through tube 39 to the dialyzer 24 for treatment by a suitable dialysate passing on the outside of the multitude of tubular membranes within which the blood is passing. The blood flows from the dialyzer into the air collection chamber 26 where it is treated with an anticoagulant fed by syringe pump 34. This pump may have its plunger connected to a slide-mounted rack operated by a pinion rotated by a stepper motor. Treated blood returns to the patient from the chamber 26 through the venous tube 37.

While the blood initially passes through the blood circuit at the start of the treatment, it displaces the dialysate in the blood circuit, and the displaced dialysate passes through the venous tube 37 into the patient. When the reservoir 30 is filled a predetermined amount corresponding to the increased liquid retention of the patient since the last dialysis treatment, the filtrate pump 28 is stopped and the pinch valve 32 is closed. If the patient shows signs of discomfort or distress because of a liquid shortage caused by the treatment, the filtrate pump 28 is started in reverse with the pinch valve 33 open so that filtrate can be reintroduced to the patient via the tube 42, air collection chamber 26, and venous tube 37 where it blends with the returning blood to the patient.

After a dialysis treatment is completed it is necessary to clean all of the system components to be reused. Then the system is sterilized with a suitable sterilant solution which is left in the system until the system is to be again activated for another treatment. At that time the sterilant is replaced by dialysate before the system is connected to the patient.

One of the advantages of the system is that the vessel 50 and related ingress and egress blood tubes 36, 38 can be removed laterally as a unit from the blood pump and pinch valve mechanisms in the blood circuit, and that the filtering tube 43 can be removed laterally from the roller pump 28 in the filtrate circuit without being disconnected. Specifically, vessel 50 can be removed from between the compression shoe 52 and wall 51; tubes 36 and 38 can be removed from between pinch elements 62, 63 and blocks 64, 65; the tubes 41, 42 and 44 can be removed from the pinch valves 45, 46 and 47; and the filtrate tube 43 can be removed from between the roller elements 28a and arcuate abutment plate 28b of the roller pump 28. Thus, all of the system subjected to contact with a patient's blood or filtrate during dialysis, including all tubes, valves, pressure monitors, the reservoir 30, hemofilter 22, dialyzer 24, air collection unit 26, and anticoagulant pump 34, can be removed as a module from the dialysis machine for storage to be used for subsequent dialysis treatments of the same patient. Furthermore, this module can be cleaned, tested, and sterilized on a processing machine, thereby freeing the dialysis machine for use on another patient after inserting of that patient's module. This processing machine preferably includes reversible pumps and pinch valve mechanisms arranged to function on the vessel 50, blood tubes 36, 38 and filtrate tubes 41, 42, and 43 by a squeezing action in a suitable manner like the blood pump 18 and its related pinch valves 19-20, and filtrate pump 28 and pinch valves 32, 33 and 46.

Since post-treatment operations on the system will be basically the same whether or not performed on another machine, the following example will be with reference to the machine previously described.

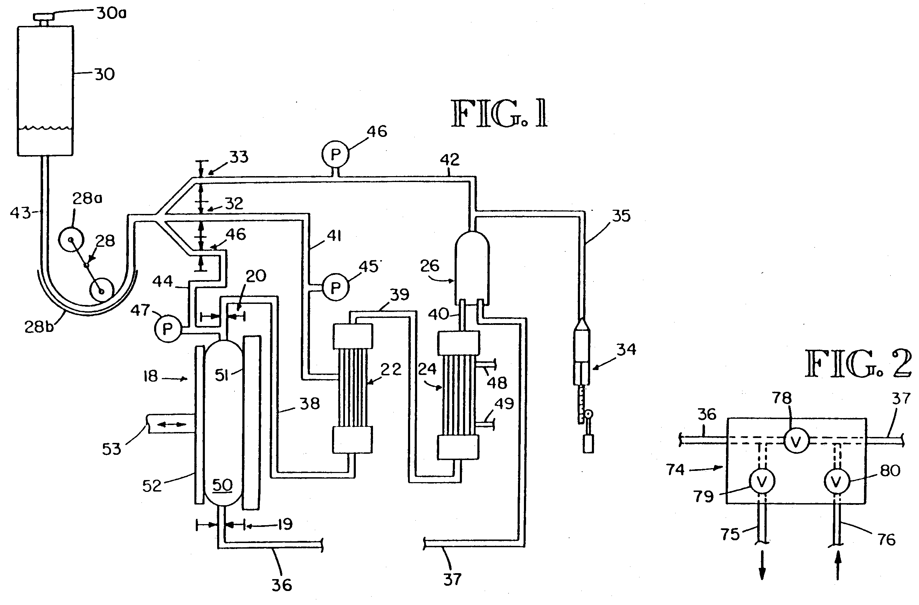

After dialysis is complete and the blood in the blood circuit has been returned to the patient and tubes 36, 37 have been disconnected from the patient, it is preferred to connect them to a valve block 74 (Figure 2) having four ports, one for the arterial tube 36, a second for the venous tube 37, a third for a drain tube 75, and a fourth for an infeed tube 76. The block 58 contains a cross-over valve 78 for connecting the tubes 36, 37 and respective valves 79, 80 for controlling flow through the drain and infeed tubes 75, 76.

To clean blood residue from the system it is necessary to flush and back-wash the components several times with a suitable flushing solution which will pass through the pores in the multitude of tubular membranes in the filter 22 and dialyzer 24. The flushing solution is preferably introduced to the system through the dialysate infeed port 48 of the dialyzer 24 and the infeed tube 76 to the block 74.

The system can initially be drained by opening the drain valve 79 and cross-over valve 78, and reversing the filtrate pump 28 and blood pump 18 for down-flow with the pinch valves 32, 33 and 46 open. Then the system can be filled with a suitable cleaning solution such as a weak household bleach through the infeed tube 76 with valve 79 closed and the filtrate pump 28 and blood pump 20 reversed for up-flow. The hemodialyzer 24 can be back-washed by introducing cleaning solution through the dialysate intake 48. Repeatedly draining and filling of the system with the cleaning solution and then with filtered water cleans out all blood and blood filtrate residue from the system components. Then the system is filled with a suitable sterilant such as formaldehyde.

When the system is to be used again, the sterilant is removed from the system, preferably by dialysis. Dialysate is circulated through the dialyzer 24 with the arterial and venous tubes 36, 37 connected together via valve 78 with valves 79, 80 being closed, and the blood pump 18 operating in up-flow mode. The filtrate pump 28 is operated in the up-flow direction with the pinch valves 32, 33 and 46 open to fill the reservoir 30, and then is reversed. This sequence is repeated until all of the sterilant has been purged from the system through the dialyzer membrane and dialysate discharge port 49. During the procedure the anticoagulant pump 34 is operated a sufficient time to remove sterilant from the connecting tube between the pump 34 and the air collection chamber 26. When the sterilant has been removed by dialysis in preparation for patient treatment, the system contains sterile dialysate.

The invention has been described with respect to a single blood pump 18. However, as shown in Figure 4, the blood pump mechanisms is ideal for operating two blood pumps 18, 18' in alternating cycles. In the two-pump arrangement the slide rod 53 is connected to a second compression shoe 52' operating with a second squeeze chamber 50'. This second chamber connects at its ends with ingress and egress tube branches 36', 38' which pass between the pinch elements 62, 63 and a second set of stationary blocks 64', 65'. A second microswitch 70' is operated in response to expansion of the chamber 50' against a wall 51' and second button 68' by way of an adjustment screw 71' and swing arm 69' which is biased by a spring 72'.

By the described two-pump system while one of the chambers 50, 50' is being compressed to discharge blood, the other chamber is being filled from the patient. Feedback from one squeeze chamber to the other is prevented by the fact that the squeeze elements 62, 63 alternatively pinch the ingress and egress tubes for the chambers. The electrical circuit from the blood pump motor to the switches 70, 70' is such that the motor can only be activated by alternate filling of the squeeze chambers 50, 50'.

The pressure sensor 47 makes it possible to monitor the output pressure of the blood pump(s), and the pressure sensors 45, 46 enable pressure monitoring of the filtrate circuit. It will be appreciated that the described system can be controlled by an automated electrical control system connected to the motor for the blood pump(s) and filtrate pump 28, and to solenoids operating the pinch elements of the valves 32, 33 and 46. It will also be appreciated that the ingress and egress pinch valves 19, 20 can have their pinch elements 62, 63 solenoid operated, and that other mechanisms can be used to operate the compression shoes 52, 52'.

## Claims

1. A hemodialysis system comprising:
a hemodialyzer (24);
a blood pump (18) comprising at least one flexible vessel (50,50');
said at least one flexible vessel (50,50') being adapted to self expand to its original shape after being deformed by compression;
a blood circuit for passing blood from a patient to said vessel (50) and from said vessel to the hemodialyzer (24), and for returning blood from the hemodialyzer to the patient, said blood circuit having an ingress valve (19) between said vessel and the patient, and an egress valve (20) between said vessel and the hemodialyzer;
a cycling means (51-56) for alternately filling and emptying said vessel in alternating pumping and suction strokes, for opening said ingress valve and closing said egress valve when said vessel is to be filled, and for closing the ingress valve and opening the egress valve when said vessel is to be emptied;
characterized by
means (68-71) for regulating said cycling means (51,56) including a sensor (68) normally contacting said vessel (50) and adapted to deactivate said cycling means (51-56) during said suction stroke whenever the suction stroke progresses beyond the extension of the vessel to an extent breaking contact of the sensor (68) with the vessel, such that the blood pump (18) is regulated by the supply of blood from the patient.

2. A hemodialysis system according to claim 1 comprising flexible ingress and egress tubes (36,38) at opposite ends of the flexible vessel (50), which extend through pinching devices (63,65;62,64) functioning together with the tubes (36,38) as ingress and egress pinch valves (19,20);
a hemofilter (22) having a blood inlet connected to said blood outlet of the pump, and having a blood outlet and a filtrate outlet;
said hemodialyzer (24) having a blood outlet, a blood inlet connected to said blood outlet of the hemofilter, and a dialysate inlet (48) and outlet (49); a filtrate reservoir (30);
an air-removal and filtrate pump (23) having an outlet connected to said reservoir and having an inlet;
an air collection chamber (26) having a blood inlet connected to said blood outlet of the hemodialyzer (24), a blood outlet for connection to a patient via a venous route (37), and an air removal outlet;
tube and valve means selectively connecting and disconnecting said inlet of the air-removal and filtrate pump with said filtrate outlet of the hemofilter and air removal outlets; and
means (34,35) for introducing an anti-coagulant to said air collection chamber (26).

3. A hemodialysis system according to claim 2 in which said filtrate pump (28) is selectively reversible to pump filtrate from said reservoir (30) to the hemofilter (22) and/or air collection chamber (26) to enable return of the blood in the system to the patient via the arterial and venous routes (36,37) at the close of a hemodialysis treatment.

4. A hemodialysis system according to claim 2 in which additional tube and valve means (43,44,46) selectively connect said blood outlet of said blood pump (18) with said reservoir (30).

5. A hemodialysis system according to claim 1 wherein said cycling means comprises compression means (51-56) for selectively collapsing said at least one vessel during a compression stroke to discharge blood into said egress tube when said egress tube is open and said ingress tube is collapsed, said compressing means also having a return stroke permitting said vessel (50) to return to its original shape to create a suction in said ingress tube when said egress tube is collapsed and said ingress tube is open, said valve mechanisms (19,20) being connected to said compression means for operating in a cycle wherin said ingress tube is open and said egress tube is closed during said return stroke, and wherein said ingress tube (36) is closed and said egress tube (38) is open during said compression stroke, wherein said valve mechanisms (19,20) are pinch valves and wherein said compression means includes a rotary combination crank and cam member (55) a compression shoe (52) engaging said vessel, and a link (53) pivotally connected to said member and shoe for linearly reciprocating said shoe responsive to turning of said crank and cam member (55); and in which said pinch valve mechanisms include rockers (60,61) with pinch elements (62,63) at one end of the rockers and cam followers (58,59) at the other end thereof engaging said, crank and cam member while it turns.

6. A hemodialysis system according to claim 5 in which said ingress tube (36) is collapsed before said egress tube (38) is permitted to open during each cycle.

## Patentansprüche

1. Dialysesystem, das umfaßt:
ein Dialysegerät (24);
eine Blutpumpe (18), die wenigstens ein flexibles Gefäß (50,50') umfaßt;
wobei das wenigstens eine flexible Gefäß (50,50') sich von selbst in seine ursprüngliche Form ausdehnt, nachdem es durch Zusammendrücken verformt worden ist;
einen Blutkreislauf, der Blut von einem Patienten zu dem Gefäß (50) und von dem Gefäß zu dem Dialysegerät (24) leitet und Blut von dem Dialysegerät zu dem Patienten zurückführt, wobei der Blutkreislauf ein Eintrittsventil (19) zwischen dem Gefäß und dem Patienten sowie ein Austrittsventil (20) zwischen dem Gefäß und dem Dialysegerät aufweist;
eine Takteinrichtung (51-56), die das Gefäß in abwechselnden Pump- und Ansaughüben abwechselnd füllt und leert, und das Eintrittsventil öffnet und das Austrittsventil schließt, wenn das Gefäß gefüllt werden soll, und das Eintrittsventil schließt und das Eintrittsventil öffnet, wenn das Gefäß geleert werden soll; **gekennzeichnet durch**
eine Einrichtung (68-71), die die Takteinrichtung (51,56) regelt und einen Sensor (68) enthält, der normalerweise mit dem Gefäß (50) in Kontakt ist und die Takteinrichtung (51-56) während des Ansaughubes immer dann außer Funktion setzt, wenn der Ansaughub die Ausdehnung des Gefäßes in einem Maße überschreitet, bei dem der Kontakt des Sensors (68) mit dem Gefäß unterbrochen wird, so daß die Blutpumpe (18) durch die Zufuhr von Blut von dem Patienten geregelt wird.

2. Dialysesystem nach Anspruch 1, das flexible Eintritts- und Austrittsröhren (36,38) an einander gegenüberliegenden Enden des flexiblen Gefäßes (50) umfaßt, die durch Quetschvorrichtungen (63,65;62,64) hindurch verlaufen, die zusammen mit den Röhren (36,38) als Eintritts- und Austritts-Quetschventile (19,20) wirken;
einen Blutfilter (22) mit einem Bluteinlaß, der mit dem Blutauslaß der Pumpe verbunden ist, und einem Blutauslaß sowie einem Filtratauslaß;
wobei das Dialysegerät (24) einen Blutauslaß aufweist; einen mit dem Blutauslaß des Blutfilters verbundenen Bluteinlaß und einen Dialysateinlaß (48) und -auslaß (49); sowie einen Filtratbehälter (30);
eine Luftabführ- und Filtratpumpe (28) mit einem Auslaß, der mit dem Behälter verbunden ist, und einem Einlaß;
eine Luftauffangkammer (26) mit einem Bluteinlaß, der mit dem Blutauslaß des Dialysegeräts (24) verbunden ist, einem Blutauslaß zum Anschluß an einen Patienten über einen Venenweg (37) sowie einem Luftabführauslaß;
Röhren- und Ventileinrichtungen, die den Einlaß der Luftabführ- und Filtratpumpe wahlweise mit dem Filtratauslaß des Dialysegeräts und Luftabführauslässen verbinden bzw. sie davon trennen; und
eine Einrichtung (34,35), die ein Antikoagulans in die Luftsammelkammer (26) einleitet.

3. Dialysesystem nach Anspruch 2, wobei die Filtratpumpe (28) wahlweise umkehrbar ist, um Filtrat aus dem Behälter (30) zu dem Blutfilter (22) und/oder der Luftauffangkammer (26) zu pumpen und Rückkehr des Blutes in dem System zum Patienten über die Arterien- und Venenwege (36,37) beim Abschluß der Dialysebehandlung zu ermöglichen.

4. Dialysesystem nach Anspruch 2, wobei zusätzliche Röhren- und Ventileinrichtungen (43,44,46) den Blutauslaß der Blutpumpe (18) wahlweise mit dem Behälter (30) verbinden.

5. Dialysesystem nach Anspruch 1, wobei die Takteinrichtung eine Zusammendrückeinrichtung (51-56) umfaßt, die das wenigstens eine Gefäß während eines Zusammendrückhubes wahlweise zusammendrückt, um Blut in die Austrittsröhre abzugeben, wenn die Austrittsröhre offen und die Eintrittsröhre zusammengedrückt ist, wobei die Zusammendrückeinrichtung darüber hinaus einen Rückkehrhub aufweist, der es dem Gefäß (50) ermöglicht, in seine ursprüngliche Form zurückzukehren und einen Sog in der Eintrittsröhre zu erzeugen, wenn die Austrittsröhre zusammengedrückt und die Eintrittsröhre geöffnet ist, wobei der Ventilmechanismus (19,20) mit der Zusammendrückeinrichtung verbunden ist, um in einem Zyklus zu arbeiten, bei dem während des Rückkehrhubes die Eintrittsröhre geöffnet und die Austrittsröhre geschlossen ist, und bei dem während des Zusammendrückhubes die Eintrittsröhre (36) geschlossen und die Austrittsröhre (38) geöffnet ist, wobei es sich bei den Ventilmechanismen (19,20) um Quetschventile handelt, und wobei die Zusammendrückeinrichtung ein sich drehendes, aus Kurbel und Nocken kombiniertes Element (55) enthält, eine Druckplatte (52), die mit dem Gefäß in Kontakt ist, und eine Verbindungsstange (53), die drehbar mit dem Element und der Platte verbunden ist, um die Platte beim Drehen des Kurbel-und-Nocken-Elementes (55) linear hin- und herzubewegen; und wobei die Klemmventilmechanismen Kipphebel (60,61) mit Quetschelementen (62,63) an einem Ende der Kipphebel und Nockeneingriffsgliedern (58,59) am anderen Ende derselben enthalten, die mit dem Kurbel-und-Nocken-Element in Eingriff sind, während es sich dreht.

6. Dialysesystem nach Anspruch 5, wobei die Eintrittsröhre (36) zusammengedrückt wird, bevor sich die Austrittsröhre (38) während jedes Zyklus öffnen kann.

## Revendications

1. Système d'hémodialyse comprenant :
un dispositif d'hémodialyse (24) ;
une pompe de sang (18) comprenant au moins un récipient flexible (50, 50') ;
ledit au moins un récipient flexible (50, 50') étant propre à se dilater pour retrouver sa forme initiale après avoir été déformé par compression ;
un circuit de sang pour amener du sang d'un patient vers ledit récipient (50) et dudit récipient jusqu'au dispositif d'hémodialyse (24), et pour ramener le sang du dispositif d'hémodialyse vers le patient, ledit circuit de sang comportant une vanne d'entrée (19) entre ledit récipient et le patient, et une vanne de sortie (20) entre ledit récipient et le dispositif d'hémodialyse ;
des moyens de recyclage (51, 56) pour alternativement remplir et vider ledit récipient par battements alternatifs de pompage et d'aspiration, pour ouvrir ladite vanne d'entrée et fermer ladite vanne de sortie quand ledit récipient doit être rempli, et pour fermer la vanne d'entrée et ouvrir la vanne de sortie quand ledit récipient doit être vidé ;
caractérisé par des moyens (68-71) pour réguler ledit moyen de recyclage (51, 56), comprenant un capteur (68) normalement en contact avec le récipient (50) et propre à désactiver lesdits moyens de recyclage (51-56) pendant ledit battement d'aspiration lorsque le battement d'aspiration progresse au-delà d'une position d'expansion du récipient où le contact du capteur (68) avec le récipient est interrompu, de manière que la pompe de sang (18) est régulée par la fourniture de sang du patient.

2. Système d'hémodialyse selon la revendication 1, comprenant des tubes flexibles d'entrée et de sortie (36, 38) à des extrémités opposées du récipient flexible (50), qui s'étendent à travers de dispositifs de pincement (63, 65 ; 62, 64) coopérant avec les tubes (36, 38) pour former des vannes d'entrée et de sortie à pincement (19, 20) ;
un filtre de sang (22) ayant une entrée de sang reliée à la sortie de sang de la pompe, et ayant une sortie de sang non-filtré et une sortie de sang filtré ;
ledit dispositif d'hémodialyse (24) ayant une sortie de sang non-dialysé, une entrée de sang reliée à ladite sortie de sang non-filtré du filtre de sang, et une entrée (48) et une sortie (49) de sang dialysé ;
un réservoir de sang filtré (30) ; une pompe (28) d'extraction d'air et de sang filtré ayant une sortie reliée audit réservoir et ayant une entrée,
une chambre de rassemblement d'air (26) ayant une entrée de sang reliée à ladite sortie de sang non-dialysé du dispositif d'hémodialyse (24), une sortie de sang à relier à un patient par une voie veineuse (37), et une sortie d'extraction d'air ;
des moyens de tube et de vanne connectant et déconnectant sélectivement ladite entrée de la pompe d'extraction d'air et de sang filtré avec ladite sortie de sang filtré du filtre de sang et la sortie d'extraction d'air ; et
des moyens (34, 35) pour introduire un anticoagulant dans ladite chambre de rassemblement (26).

3. Système d'hémodialyse selon la revendication 2, dans lequel ladite pompe de sang filtré (28) est sélectivement réversible pour pomper du sang filtré à partir dudit réservoir (30) jusqu'au filtre de sang (22) et/ou jusqu'à la chambre de rassemblement d'air (26) pour permettre un retour du sang du système vers le patient par les voies artérielle et veineuse (36, 37) à la fin du traitement d'hémodialyse.

4. Système d'hémodialyse selon la revendication 2, dans lequel des moyens supplémentaires de tube et de vanne (43, 44, 46) relient sélectivement ladite sortie de sang non-filtré de ladite pompe de sang (18) audit réservoir (30).

5. Système d'hémodialyse selon la revendication 1, dans lequel lesdits moyens de recyclage comprennent des moyens de compression (51-56) pour sélectivement écraser ledit au moins un récipient pendant un battement de compression pour décharger du sang dans ledit tube de sortie quand ledit tube de sortie est ouvert et ledit tube d'entrée est écrasé, lesdits moyens de compression présentant également un battement de retour pour permettre audit récipient (50) de retrouver sa forme initiale pour provoquer une aspiration dans ledit tube d'entrée quand ledit tube de sortie est écrasé et ledit tube d'entrée est ouvert, lesdits mécanismes de vanne (19, 20) étant reliés auxdits moyens de compression pour fonctionner dans un cycle pendant lequel ledit tube d'entrée est ouvert et ledit tube de sortie est fermé lors dudit battement de retour, et dans lequel ledit tube d'entrée (36) est fermé et ledit tube de sortie (38) est ouvert pendant ledit battement de compression, les mécanismes de vanne (19, 20) étant des vannes à pincement et lesdits moyens de compression comprenant un système à manivelle et came (55), un patin de compression (52) appuyant sur ledit récipient, et un levier (53) articulé sur ladite came et le patin pour faire déplacer ledit patin en va-et-vient linéaire en réponse à la rotation dudit système à manivelle et came (55) ; et dans lequel lesdits mécanismes de vanne à pincement comportent des bras oscillants (60, 61) munis d'éléments de pincement (62, 63) à une extrémité des bras oscillants et des éléments de poursuite (58, 59) à l'autre extrémité en contact avec ledit système à manivelle et came tandis qu'il tourne.

6. Système d'hémodialyse selon la revendication 5, dans lequel ledit tube d'entrée (36) est écrasé avant de permettre l'ouverture dudit tube de sortie (38) pendant chaque cycle.
